# EUROPEAN PATENT APPLICATION

(11) **EP 4 124 378 A1**
(43) Date of publication of application: **01.02.2023**
(21) Application number: 21188301.2
(22) Date of filing: 28.07.2021
(51) Int. Cl.: B01D 61/02, B01D 61/04, B01D 61/24, B01D 61/58, B01D 61/12, A61M 1/16, C02F 1/32, C02F 1/44

(54) **WATER PURIFICATION DEVICE FOR DIALYSIS**

(71) Applicant: Greentec Dialysis GmbH, 69115 Heidelberg (DE)
(72) Inventor: The designation of the inventor has not yet been filed
(74) Representative: Maiwald GmbH

(57) **Abstract**

A purification device (100) for generating purified water suppliable to a dialysis device (300) is proposed. The purification device comprises at least one inlet (102) configured to receive inlet water from at least one water source (200), a reverse osmosis apparatus (120) fluidly coupled to the at least one inlet (102) and configured to purify, based on reverse osmosis, the inlet water to generate purified water, at least one outlet (104) fluidly coupled to the reverse osmosis apparatus (120) and configured to supply the purified water to a dialysis device (300) for dialysis treatment, and a heating system (110) configured to heat the inlet water received by the at least one inlet (102) and supplied to the reverse osmosis apparatus (120).

## Description

### Technical Field

The present disclosure generally relates to the field of dialysis. In particular, the present disclosure relates to a purification device for generating purified water suppliable to a dialysis device. Further, the present disclosure relates to a purification system comprising such purification device and at least one dialysis device.

### Technical Background

Generally, dialysis is used to purify blood from patients whose kidneys may be functionally disturbed and/or may have failed. In dialysis, the blood to be purified is extracted from a patient and supplied to a dialysis device, where the blood usually passes a semipermeable membrane on one side of the membrane with a sterile solution flowing on another side of the membrane. The semipermeable membrane is also referred to as dialyzer. Due to a gradient in concentration of substances and/or constituents in the fluids on the two sides of the membrane, an osmotic pressure is generated that can cause certain substances and/or constituents to pass from the blood through the semipermeable membrane into the solution. Typically, substances and/or constituents extracted from the patient's blood by dialysis can only hardly be removed from a patient's blood by the patient itself due to an organ failure or malfunction. However, too high concentrations of certain substances and/or constituents can be troublesome for the patient and adversely affect its health status.

Dialysis is a common treatment procedure today that can help a large number of people. Because a single dialysis treatment of a patient lasts for several hours but has to be repeated regularly, dialysis is usually performed in dedicated dialysis centers where a large number of dialysis stations with appropriate dialysis machines, devices, apparatuses and/or systems is provided.

Dialysis devices typically prepare, inter alia, the sterile solution into which the interfering substances and/or constituents are to be extracted from the blood. These dialysis solutions are typically prepared from water, in particular drinking water and/or fresh water, which was previously purified by reverse osmosis. Such purified water (also referred to as dialysis water, pure water and/or permeate) is usually heated to a body temperature of around 37°C in the dialysis device, mixed with an electrolyte, which can optionally be mixed from different solutions in the dialysis device, to create a dialysis solution with favorable, predetermined and/or defined conditions for the patient.

The amounts and quantities of water treated or prepared in this manner to supply the purified dialysis water to dialysis devices are considerably high. For this reason, dialysis centers or institutions usually use a central reverse osmosis system or water treatment system providing the purified water via respective feed lines to the dialysis devices. Such water treatment systems typically consist of one or more filters, a softening system for decalcification and a reverse osmosis system. Fresh water or drinking water is usually supplied via the one or more filters and the softening system to the reverse osmosis system, where the filtered and softened (decalcified) water can be purified to such an extent that it can be fed directly to the dialysis devices for treatment. The permeate is typically heated in the dialysis devices and electrolytes can be added to the permeate to create the dialysis solution into which substances and/or constituents can be extracted from the patient's blood via the semipermeable membrane, as described hereinabove. The used permeate is then usually fed back to the central water treatment system for purification via reverse osmosis.

The process of purification of water for dialysis and the generation of the dialysis solution can consume a considerable amount of electrical energy. Also, water consumption or usage can be considerably high.

### Summary

For the reasons set out hereinabove, it may be desirable to provide for an improved purification device for generating purified water for dialysis.

This is achieved by the subject matter of the independent claims, wherein further embodiments are defined in the dependent claims and the following description.

According to an aspect of the present disclosure, there is provided a purification device for generating purified water, permeate and/or pure water suppliable to a dialysis device, a dialysis apparatus, a dialysis machine and/or a dialysis system. The purification device comprises at least one inlet configured to receive inlet water from at least one water source, and a reverse osmosis apparatus fluidly coupled to and/or in fluid communication with the at least one inlet, the reverse osmosis apparatus being configured to purify, based on reverse osmosis, the inlet water to generate purified water. The purification device further comprises at least one outlet fluidly coupled to and/or in fluid communication with the reverse osmosis apparatus, the at least one outlet being configured to supply the purified water to a dialysis device for dialysis treatment. The purification device further comprises a heating system configured to heat the inlet water received by the at least one inlet and supplied to the reverse osmosis apparatus. In particular, the heating system may be configured to heat the inlet water, such that a temperature of inlet water supplied to the reverse osmosis apparatus, as well as a temperature of the outlet water drained from the purification device and/or supplied to the dialysis device via the outlet exceeds a temperature of the inlet water supplied to and/or received by the purification device via the inlet.

Accordingly, inlet water may be received via the inlet and heated by the heating system to supply the heated and/or pre-heated inlet water to the reverse osmosis apparatus of the purification device to generate the purified water. Since an efficiency or performance of the reverse osmosis apparatus, which can be given as amount of purified water generated per amount of inlet water supplied to the reverse osmosis apparatus, is proportional to and/or depends on a temperature of the inlet water to be purified, heating the inlet water prior to reverse osmosis and/or prior to the purification based on reverse osmosis can significantly improve the efficiency or performance of the reverse osmosis process. In other words, more purified water can be generated per amount of inlet water.

Moreover, since also the outlet water drained from the purification device and/or supplied to the dialysis device has a higher temperature, e.g. when compared to a temperature of the inlet water received from the at least one water source, an energy consumption of the dialysis device can be significantly reduced, because the dialysis device may not have to heat the received outlet water as much to reach the desired body temperature of about 37°C. In particular, the outlet water may be heated by the heating system of the purification device even to a temperature sufficiently high, such that no heating may be required at the dialysis device. Hence, the purification device according to present disclosure can significantly improve an overall efficiency of the water purification process and/or the dialysis treatment, in particular allowing for a significant reduction in power consumption.

As used herein, the at least one water source can refer to a supply of water, in particular drinking water or fresh water. The at least one water source can, for example, be coupled to a drinking water supply network. Alternatively or additionally, the at least one water source may comprise one or more containers or tanks for supplying the inlet water to the purification device. When referring to a single water source hereinabove or hereinbelow, a plurality of water sources is included.

The at least one inlet of the purification device may be fluidly coupled to the at least one water source by appropriate tubing or piping. Similarly, the at least one outlet of the purification device may be fluidly coupled to the at least one dialysis device by appropriate piping or tubing. When referring to a single inlet and/or outlet hereinabove and hereinbelow, also a plurality of inlets and/or outlets is included.

According to an embodiment, at least a part of the heating system is arranged between the at least one inlet and the reverse osmosis apparatus. Alternatively or additionally, at least a part of the heating system is arranged upstream of the reverse osmosis apparatus. As used herein, the terms upstream and downstream may refer to a stream of water within the device or system, for example from the at least one water source, via the inlet through the purification device and from the outlet to the dialysis device.

In an example, the heating system may comprise one or more heating elements arranged upstream of the reverse osmosis apparatus and configured to heat the inlet water from an initial temperate of the inlet water to a predefined and/or predetermined temperature above the initial temperature to generate heated or pre-heated water that can be supplied to the reverse osmosis apparatus.

According to an embodiment, the heating system is configured to heat the inlet water by at least 10%, preferably by at least 25% of a temperature of the inlet water at the at least one inlet, which may be referred to herein as initial temperature. For example, the heating system may be configured to heat the inlet water by at least 5%, preferably at least 10%, even more preferably at least 25%, at least 50% or even more relative to the initial temperature of the inlet water when received at the inlet. This may allow to significantly improve the efficiency of the reverse osmosis apparatus, while allowing to reduce an overall energy consumption, as described hereinabove.

According to an embodiment, the heating system is configured to heat the inlet water to a temperature of at least 15°C, preferably at least 20°C. It should be noted that the heating system may be configured to heat the inlet water to a temperature substantially corresponding to the body temperature of about 37°C or even a higher temperature, such that reduced or even no heating may be required at the dialysis device.

According to an embodiment, the heating system is configured to heat the inlet water to a maximum temperature of about 25°C.

Generally, a microbial load of a dialysis water system, a purification system and/or a water treatment system may be a quality factor that may be regulated according to national or international standards. As a microbial load in the dialysis water, for example the inlet water, the purified water and/or the outlet water, may increase with temperature, it may be favorable to limit the temperature of the inlet water supplied to the reverse osmosis apparatus and/or the temperature of the outlet water supplied to the dialysis device to a certain predefined and/or predetermined maximum temperature, such as for example 25°C. It should be noted, though, that the present disclosure is not limited to such maximum temperature. Rather, the purification device and/or the heating system thereof can be configured to heat the water to a temperature above 25°C, as described hereinabove.

According to an embodiment, the purification device is supplied with renewable energy. This may further allow to reduce a carbon footprint of the overall dialysis treatment and/or the purification process performed by means of the purification device.

According to an embodiment, the heating system includes at least one heat exchanger arranged upstream of the reverse osmosis apparatus in a supply line for supplying the inlet water to the reverse osmosis apparatus. The supply line may refer to or include corresponding tubing or piping to convey the inlet water received at the inlet to the at least one heat exchanger and/or to the reverse osmosis apparatus. Further, the at least one heat exchanger may be driven by and/or supplied with thermal energy via a secondary circuit and/or heating circuit, which optionally can be supplied with energy from one or more renewable energy sources. This can allow for an efficient water purification at minimum carbon footprint.

According to an embodiment, the heating system is configured to heat the inlet water based on heat exchange with a heating circuit, for example a secondary circuit, wherein the heating system includes at least one heating element configured to heat a heat exchange fluid of the heating circuit. In other words, a heating circuit may be provided as secondary circuit of the heating system, wherein the heat exchange fluid can be heated by the at least one heating element. The heating circuit and/or the heating system may further be configured to heat the inlet water, which may flow in a primary circuit of the purification device based on transferring thermal energy from the heat exchange fluid to the inlet water, for example based on convection and/or based on thermal coupling.

According to an embodiment, the heating system includes at least one pump arranged in a heating circuit of the heating system, wherein the at least one pump is controllable to adjust a flow rate of a heat exchange fluid in the heating circuit. By controlling the flow rate of the heat exchange fluid, the temperature of the heat exchange fluid and in turn the temperature of the inlet water (or heated inlet water) supplied to the reverse osmosis apparatus can be precisely controlled.

According to an embodiment, the purification device further comprises a bypass line with one or more controllable valves, wherein the bypass line is arranged upstream of the reverse osmosis apparatus and configured to bypass the heating system to supply non-heated inlet water to the reverse osmosis apparatus. Via the bypass line and the one or more controllable valves at least a part of or the entire inlet water received at the inlet may bypass the heating system. When only a part of the inlet water is bypassed, the bypassed water may optionally be mixed with the heated inlet water supplied from the heating system via the supply line to the reverse osmosis apparatus in order to supply the mixed water at a desired temperature to the reverse osmosis apparatus. This can allow for a fast, efficient, reliable and precise temperature control.

According to an embodiment, the purification device further comprises a controller for controlling the heating system and/or a flow rate of inlet water to control a temperature of the purified water and/or of the inlet water supplied to the reverse osmosis apparatus. Generally, the controller may refer to a control circuitry comprising one or more processors. The controller may for example be configured to control or regulate a temperature of a heat exchange fluid of the heating system, to operate or control at least one heating element of the heating system, and/or to operate or control one or more control valves, for example one or more valves in a bypass line and/or a heating circuit. Alternatively or additionally, the controller may be configured to control the reverse osmosis apparatus and/or one or more other components or elements of the purification device. Optionally, the controller may receive one or more sensor signals of one or more sensors of the purification device, such as temperature sensors, flow rate sensors and/or pressure sensors, to control and/or adjust the temperature of the inlet water supplied to the reverse osmosis apparatus.

In an example, the controller may be configured to control and/or adjust a temperature of the inlet water supplied to the reverse osmosis apparatus to a predetermined and/or predefined temperature. Such predefined and/or predetermined temperature may be stored in a memory or data storage of the purification device or may be retrieved from an external data source.

According to an embodiment, the purification device further comprises at least one temperature sensor arranged upstream of at least a part of the heating system and configured to determine a temperature of the inlet water received from the at least one inlet. Alternatively or additionally, the purification device further comprises at least one temperature sensor arranged downstream of the heating system (or at least a part thereof) and configured to determine a temperature of the inlet water supplied to the reverse osmosis apparatus. Using one or more temperature sensors arranged upstream and/or downstream of the heating system can allow for a fast and precise temperature control.

For example, one or more sensor signals or sensor readings of the one or more temperature sensors may be processed by the controller to determine a temperature of the inlet water supplied to the reverse osmosis apparatus. The controller may further be configured to determine a deviation of the determined temperature from a predefined and/or predetermined temperature. Based on the determined deviation, the controller may be configured to adjust and/or control the temperature of the inlet water supplied to the reverse osmosis apparatus, such that the deviation from the predefined and/or predetermined temperature reaches or is below a threshold value for the deviation.

According to an embodiment, the purification device further comprises at least one flow rate sensor arranged upstream of the reverse osmosis apparatus and configured to determine a flow rate of inlet water supplied to the reverse osmosis apparatus. Alternatively or additionally, the purification device comprises at least one flow rate sensor arranged downstream of the reverse osmosis apparatus and configured to determine a flow rate of purified water supplied to the at least one outlet. Using one or more flow rate sensors arranged upstream and/or downstream of the reverse osmosis apparatus can allow to control or regulate the flow of water through the reverse osmosis apparatus.

For example, one or more sensor signals or sensor readings of the one or more flow rate sensors may be processed by the controller to determine the flow rate of water through the reverse osmosis apparatus. The controller may further be configured to determine a deviation of the determined flow rate from a predefined and/or predetermined flow rate. Based on the determined deviation, the controller may be configured to adjust and/or control the flow rate of water through the reverse osmosis apparatus, such that the deviation from the predefined and/or predetermined flow rate reaches or is below a threshold value for the deviation in flow rate.

According to an embodiment, the purification device further comprises at least one temperature control valve configured to control a temperature of the inlet water heated by the heating system based on adjusting a flow rate of non-heated inlet water supplied to the heating system or at least a part thereof. Therein, the temperature control valve may refer to or denote a valve that can be controlled based on and/or in dependence of a temperature of the inlet water supplied to the reverse osmosis apparatus. For example, the controller may be configured to actuate and/or operate the temperature control valve based on one or more sensor signals of one or more temperature sensors, such that the temperature of the inlet water supplied to the reverse osmosis apparatus substantially corresponds to or matches the predefined and/or predetermined temperature. This can include comparing a temperature determined based on the one or more temperature sensors to the predetermined and/or predefined temperature, as described hereinabove.

According to an embodiment, the purification device further comprises a sterilization device arranged upstream of the reverse osmosis apparatus between the reverse osmosis apparatus and the heating system, wherein the sterilization device is configured to sterilize inlet water supplied to the reverse osmosis apparatus. For example, the sterilization device can be configured to sterilize the inlet water supplied to the reverse osmosis apparatus based on irradiating the inlet water with UV radiation. However, other means for sterilization are also envisaged by the present disclosure. Generally, using a sterilization device can allow to reduce a microbial load of the inlet water supplied to the reverse osmosis apparatus, which in turn can increase an efficiency of the reverse osmosis process.

A further aspect of the present disclosure relates to purification system for generating purified water for dialysis treatment. The purification system comprises one or more purification devices, as described hereinabove and hereinbelow. The purification system further includes one or more dialysis devices fluidly coupled to the at least one outlet of the at least one purification device and configured to receive the purified water from the at least one purification device to generate a permeate and/or sterile solution for dialysis treatment.

It should be noted that any feature, function, element, technical effect and/or advantage described hereinabove and hereinbelow with reference to one aspect of the present disclosure, equally applies to any other aspect of the present disclosure. In particular, any feature, function, element, technical effect and/or advantage described hereinabove and hereinbelow with reference to the purification device equally applies to the purification system, and vice versa.

Yet a further aspect of the present disclosure relates to the use of a purification device and/or a purification system, as described hereinabove and hereinbelow, for generating purified water for dialysis.

A further aspect of the present disclosure relates to a method of operating a purification device and/or a purification system, as described hereinabove and hereinbelow. The method comprises:
- receiving, from an inlet of the purification device, inlet water from at least one water source;
- heating the inlet water with a heating system of the purification device;
- supplying the heated or pre-heated inlet water to a reverse osmosis apparatus of the purification device; and
- purifying the inlet water based on reverse osmosis using the reverse osmosis apparatus.

The method may optionally comprise a step of supplying the purified water via an outlet to a dialysis device.

These and other aspects of the invention will be apparent from and elucidated with reference to the appended figures, which may represent exemplary embodiments.

### Brief Description of the Drawings

The subject-matter of the invention will be explained in more detail in the following with reference to exemplary embodiments which are illustrated in the attached drawings, wherein:
Fig. 1 shows a purification system with a purification device according to an exemplary embodiment;
Fig. 2 shows a purification system with a purification device according to a further exemplary embodiment; and
Fig. 3 shows a detailed view of a part of the purification device of Fig. 2.

The figures are schematic only and not true to scale. In principle, identical or like parts are provided with identical or like reference symbols in the figures.

### Detailed Description of Exemplary Embodiments

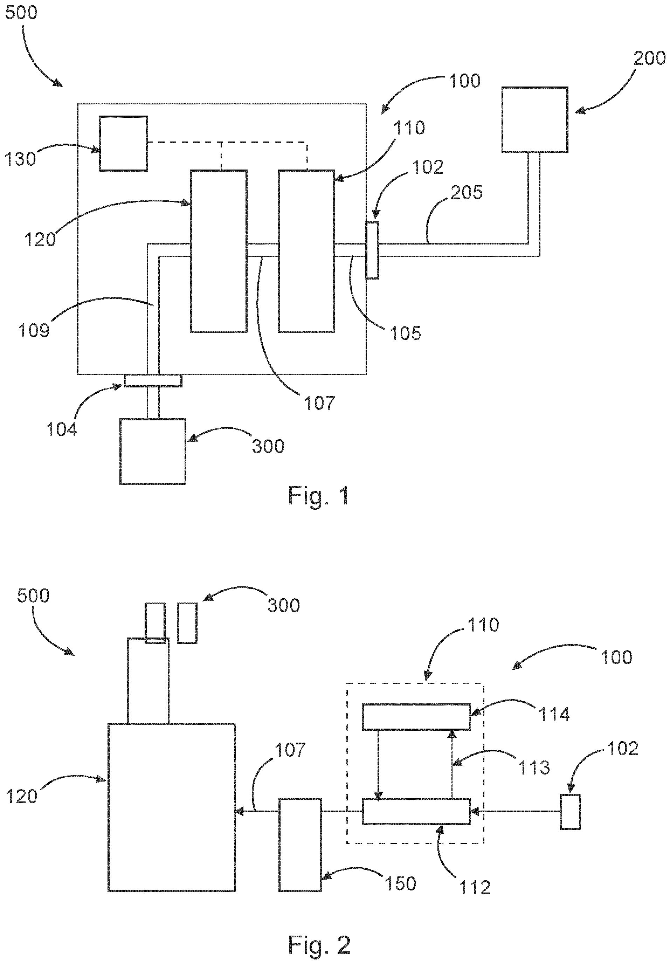

Figure 1 shows a purification system 500 with a purification device 100 for generating purified water for dialysis according to an exemplary embodiment.

The purification system 500 comprises one or more water sources 200 for supplying drinking water or fresh water to the purification device 100. The purification system 500 further comprises one or more dialysis devices 300 receiving purified water from the purification device 100, as discussed in more detail hereinabove and hereinbelow.

Therein, water is supplied from the at least one water source 200 as "inlet water" to an inlet 102 of the purification device 100. The inlet 102 and the at least one water source 200 may be fluidly coupled together and/or interconnected by a tubing, piping and/or feed line 205. The inlet water received at the inlet 102 may have an initial temperature, for example a temperature below 25°C, e.g. between about 1°C and 23°C.

The purification device 100 further comprises a heating system 110 fluidly coupled and/or interconnected with the inlet 102, for example via a corresponding piping, tubing and/or feed line 105. The heating system 110 is configured to heat the inlet water received via the inlet 102 and the feed line 105 to a temperature above the initial temperature. For example, the heating system 110 may be configured to heat the inlet by at least 5%, at least 10%, preferably by at least 25% or even more with respect to a temperature of the inlet water at the at least one inlet 102, i.e. the initial temperature. The heating system 110 may be configured to heat the inlet water to a temperature of at least 15°C, preferably at least 20°C.

Optionally, the heating system 110 may be configured to heat the inlet water to a maximum temperature of about 25°C. Higher temperatures are possible, though.

The purification device 100 further comprises a reverse osmosis apparatus 120 fluidly coupled to and/or interconnected, for example via a supply line 107, with the heating system 110. Accordingly, the heating system 110 or at least a part thereof is arranged upstream of the reverse osmosis apparatus 120, such that heated inlet water is supplied from the heating system 110 to the reverse osmosis apparatus 120. By heating the inlet water and supplying the heated inlet water to the reverse osmosis apparatus 120, an efficiency and performance of the reverse osmosis apparatus 120 can be increased, allowing to generate more purified water per amount of inlet water at reduced energy consumption.

The reverse osmosis apparatus 120 is configured to process and/or treat the heated inlet water received from the heating system 110 and purify the received water based on reverse osmosis to generate purified water.

The purified water can then be supplied via a drain line 109 of the purification device 100 to an outlet 104 thereof. The outlet 104 is fluidly coupled to and/or interconnected with the dialysis device 300, such that the purified water can be supplied to the dialysis device 300.

As noted above, since the inlet water received at the inlet 102 is heated by the heating system 110, also the purified water generated by the reverse osmosis apparatus 120 has an elevated temperature. This may allow to reduce energy consumption of the dialysis device 300 as less or even no heating may be required at the dialysis device 300 to reach a desired temperature, such as e.g. a body temperature of about 37°C.

The purification device 100 further comprises a controller 130 or control circuitry 130 configured to control a temperature of the inlet water supplied via the supply line 107 to the reverse osmosis apparatus 120, as will be described in more detail with reference to subsequent figures. The controller 130 may be configured to control one or more components of the purification device 100, such as for example the heating system 110, the reverse osmosis apparatus 120, one or more pumps, one or more valves, and/or other components.

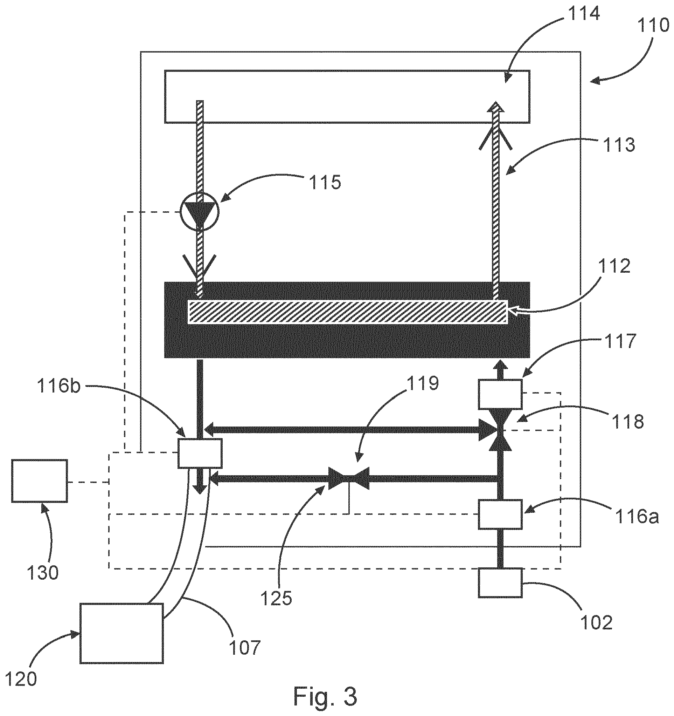

Figure 2 shows a purification system 500 with a purification device 100 according to a further exemplary embodiment. Figure 3 shows a detailed view of a part of the purification device 100 of Figure 2. Unless stated otherwise, the purification device 100 and the purification system 500 of Figures 2 and 3 comprise the same features, functions and elements as the purification device 100 or system 500 described with reference to Figure 1.

The purification device 100 shown in Figure 2 comprises a sterilization device 150 arranged upstream of the reverse osmosis apparatus 120 between the reverse osmosis apparatus 120 and the heating system 110, wherein the sterilization device 150 is configured to sterilize inlet water supplied to the reverse osmosis apparatus 120. The sterilization device 150 may be arranged in the supply line 107 and/or may be fluidly coupled with the supply line 107 to sterilize water flowing from the heating system 110 to the reverse osmosis apparatus 120. For example, the sterilization device 150 may be configured to sterilize the inlet water supplied to the reverse osmosis apparatus 120 based on irradiating the inlet water with UV radiation.

The heating system 110 of the purification device 100 shown in Figures 2 and 3 comprises at least one heat exchanger 112 arranged upstream of the reverse osmosis apparatus 120 in the supply line 107 for supplying the inlet water to the reverse osmosis apparatus 120. Accordingly, the heat exchanger 112 may fluidly coupled with the supply line 107.

The heating system 110 shown in Figures 2 and 3 is configured to heat the inlet water based on heat exchange with a heating circuit 113 or secondary circuit 113 and includes at least one heating element 114 configured to heat a heat exchange fluid of the or flowing in the heating circuit 113.

For controlling a temperature of the heat exchange fluid and consequently of the inlet water supplied to the reverse osmosis apparatus 120, the heating system 110 comprises one or more pumps 115 to adjust and/or control a flow rate of heat exchange fluid in the heating circuit 113. The one or more pumps 115 may be operatively coupled with the controller 130, such that the controller 130 can actuate or control the one or more pumps 115 to adjust the flow rate within the heating circuit 113.

Optionally, the controller 130 may also be configured to control one or more pumps for controlling a flow rate of inlet water into the heating system 110.

The heating system 110 of the purification device 100 shown in Figures 2 and 3 further comprises at least one temperature sensor 116a arranged upstream of the heat exchanger 112 and configured to determine a temperature of the inlet water received from the at least one inlet 102. The heating system 110 further comprises at least one temperature sensor 116b arranged downstream of the heat exchanger 112 and configured to determine a temperature of the inlet water supplied to the reverse osmosis apparatus 120 and/or drained from the heat exchanger 112.

The heating system 110 further comprises at least one flow rate sensor 117 arranged upstream of the heat exchanger 112, for example between the heat exchanger 112 and the inlet 102. The at least one flow rate sensor 117 is configured to determine a flow rate of inlet water supplied to the reverse osmosis apparatus 120 and/or the heat exchanger 112.

Optionally, one or more flow rate sensors may be arranged downstream of the reverse osmosis apparatus 120 to determine a flow rate of purified water supplied to the at least one outlet 104.

The heating system 110 of the purification device 100 shown in Figures 2 and 3 further comprises at least one temperature control valve 118 configured to control a temperature of the inlet water heated by the heating system 110 based on adjusting a flow rate of non-heated inlet water supplied to the heating system 110 and/or the heat exchanger 112.

The temperature sensors 116a, 116b, the flow rate sensor 117 and the temperature control valve 118 are operatively coupled to the controller 130. For controlling and/or adjusting the temperature of the inlet water drained from the heating system 110 and supplied to the reverse osmosis apparatus 120, the controller 130 can be configured to process one or more sensor signals or sensor readings of the temperature sensors 116a, 116b. The controller 130 may be configured to determine a temperature of the inlet water supplied to the reverse osmosis apparatus 120 based on the sensor readings. The controller 130 may further be configured to determine a deviation of the determined temperature from a predefined and/or predetermined temperature. Based on the determined deviation, the controller 130 may be configured to adjust and/or control the temperature of the inlet water supplied to the reverse osmosis apparatus 120, such that the deviation from the predefined and/or predetermined temperature reaches or is below a threshold value for the deviation.

For actually adjusting and/or altering the temperature of the inlet water supplied to the reverse osmosis apparatus 120, the controller 130 may be configured to actuate and/or control the one or more pumps 115 arranged in the heating circuit 113 of the heating system 110.

Alternatively or additionally, the controller 130 may be configured to modify and/or alter the flow rate of inlet water supplied to the heat exchanger 112 based on controlling the temperature control valve 118. Accordingly, the controller 130 may be configured to actuate the temperature control valve 118 in accordance with and/or based on one or more of the sensor signals from the one or more temperature sensors 116a, 116b.

As shown in the example of Figure 3, the heating system 110 further comprises a bypass line 125 with one or more controllable valves 119, wherein the bypass line 125 is arranged upstream of the reverse osmosis apparatus 120 and configured to bypass the exchanger to supply non-heated inlet water to the reverse osmosis apparatus 120. This can allow to mix heated water drained from the heat exchanger 112 with the non-heated water to adjust the temperature of the inlet water supplied to the reverse osmosis apparatus 120 to the desired, predetermined and/or predefined temperature. Therein, the one or more controllable valves 119 can be controlled by the controller 130 to adjust the temperature of the inlet water supplied to the reverse osmosis apparatus 120.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practicing the claimed invention, from a study of the drawings, the disclosure, and the appended claims.

In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A purification device (100) for generating purified water suppliable to a dialysis device (300), the purification device comprising:
at least one inlet (102) configured to receive inlet water from at least one water source (200);
a reverse osmosis apparatus (120) fluidly coupled to the at least one inlet (102) and configured to purify, based on reverse osmosis, the inlet water to generate purified water;
at least one outlet (104) fluidly coupled to the reverse osmosis apparatus (120) and configured to supply the purified water to a dialysis device (300) for dialysis treatment; and
a heating system (110) configured to heat the inlet water received by the at least one inlet (102) and supplied to the reverse osmosis apparatus (120).

2. The purification device (100) according to claim 1,
wherein at least a part of the heating system (110) is arranged between the at least one inlet (102) and the reverse osmosis apparatus (120); and/or
wherein at least a part of the heating system is arranged upstream of the reverse osmosis apparatus.

3. The purification device (100) according to any one of the preceding claims,
wherein the heating system (110) is configured to heat the inlet water by at least 10%, preferably by at least 25% of a temperature of the inlet water at the at least one inlet (102).

4. The purification device (100) according to any one of the preceding claims,
wherein the heating system (110) is configured to heat the inlet water to a temperature of at least 15°C, preferably at least 20°C.

5. The purification device (100) according to any one of the preceding claims,
wherein the heating system (110) is configured to heat the inlet water to a maximum temperature of 25°C.

6. The purification device (100) according to any one of the preceding claims,
wherein the heating system (110) includes at least one heat exchanger (112) arranged upstream of the reverse osmosis apparatus (120) in a supply line (107) for supplying the inlet water to the reverse osmosis apparatus.

7. The purification device (100) according to any one of the preceding claims,
wherein the heating system (110) is configured to heat the inlet water based on heat exchange with a heating circuit (113); and
wherein the heating system includes at least one heating element (114) configured to heat a heat exchange fluid of the heating circuit (113).

8. The purification device (100) according to any one of the preceding claims,
wherein the heating system (110) includes at least one pump (115) arranged in a heating circuit (113) of the heating system, wherein the at least one pump is controllable to adjust a flow rate of a heat exchange fluid in the heating circuit.

9. The purification device (100) according to any one of the preceding claims, further comprising:
a bypass line (125) with one or more controllable valves (119), wherein the bypass line is arranged upstream of the reverse osmosis apparatus (120) and configured to bypass at least a part of the heating system (110) to supply non-heated inlet water to the reverse osmosis apparatus (120).

10. The purification device (10) according to any one of the preceding claims, further comprising:
a controller (130) for controlling the heating system (110) and/or a flow rate of inlet water to control a temperature of the purified water and/or of the inlet water supplied to the reverse osmosis apparatus (120).

11. The purification device (100) according to any one of the preceding claims, further comprising:
at least one temperature sensor (116a) arranged upstream of at least a part of the heating system (110) and configured to determine a temperature of the inlet water received from the at least one inlet (102); and/or
at least one temperature sensor (116b) arranged downstream of the heating system and configured to determine a temperature of the inlet water supplied to the reverse osmosis apparatus (120).

12. The purification device (100) according to any one of the preceding claims, further comprising:
at least one flow rate sensor (117) arranged upstream of the reverse osmosis apparatus and configured to determine a flow rate of inlet water supplied to the reverse osmosis apparatus; and/or
at least one flow rate sensor arranged downstream of the reverse osmosis apparatus and configured to determine a flow rate of purified water supplied to the at least one outlet (104).

13. The purification device (100) according to any one of the preceding claims, further comprising:
at least one temperature control valve (118) configured to control a temperature of the inlet water heated by the heating system (110) based on adjusting a flow rate of non-heated inlet water supplied to the heating system.

14. The purification device (100) according to any one of the preceding claims, further comprising:
a sterilization device (150) arranged upstream of the reverse osmosis apparatus (120) between the reverse osmosis apparatus and the heating system (110), wherein the sterilization device (150) is configured to sterilize inlet water supplied to the reverse osmosis apparatus,
preferably wherein the sterilization device (150) is configured to sterilize the inlet water supplied to the reverse osmosis apparatus based on irradiating the inlet water with UV radiation.

15. A purification system (500) for generating purified water for dialysis treatment, the purification system comprising:
a purification device (100) according to any one of the preceding claims; and
at least one dialysis device (300) fluidly coupled to the at least one outlet of the purification device and configured to receive the purified water from the purification device to generate a permeate for dialysis treatment.
